# FASCICULE DE BREVET EUROPEEN

(11) **EP 1 625 108 B1**
(45) Date de publication et mention de la délivrance du brevet: **26.03.2008**
(21) Numéro de dépôt: 04742769.5
(22) Date de dépôt: 18.05.2004
(51) Int. Cl.: C07C 41/58, C07C 43/303, C07C 45/80, C07C 47/198

(54) **SEPARATION D'UN DIACETAL DU GLYOXAL PAR EXTRACTION LIQUIDE-LIQUIDE A CONTRE-COURANT**
TRENNUNG VON GLYOXALDIACETALEN DURCH FLÜSSIG-FLÜSSIG-GEGENSTROMEXTRAKTION
SEPARATION OF A DIACETAL OF GLYOXAL BY MEANS OF COUNTER-CURRENT LIQUID-LIQUID EXTRACTION

(30) Priorité: 22.05.2003 FR 0306168
(43) Date de publication de la demande: 15.02.2006
(73) Titulaire: Clariant Specialty Fine Chemicals (France), 92800 Puteaux (FR)
(72) Inventeur: SIMON, Olivier, F-60660 Cires-Les-Mello (FR); BLEGER, François, F-60350 Trosly Breuil (FR); SCHOUTEETEN, Alain, F-95460 Ezanville (FR)
(74) Mandataire: Ulmann, Catherine Claire
(86) Numéro de dépôt international: PCT/FR2004/001224
(87) Numéro de publication internationale: WO 2004/106274

(56) Documents cités:
- EP-A- 0 607 722
- EP-A- 0 847 976
- EP-A- 1 300 383
- GB-A- 559 362
- US-A- 2 360 959

## Description

L'invention concerne un procédé de séparation d'un diacétal du glyoxal à partir d'un mélange brut comprenant ce diacétal ainsi qu'un monoacétal du glyoxal et de l'eau, par extraction liquide-liquide à contre-courant.

Les acétals du glyoxal sont des synthons intéressants pour la synthèse organique (demande WO-A-02/42524) et peuvent être utilisés comme additifs (demandes EP-A-0 855 436, EP-A-0 512 501).

La méthode la plus répandue de préparation des diacétals du glyoxal est l'acétalisation du glyoxal par un monoalcool R-OH avec une catalyse acide selon le schéma suivant :

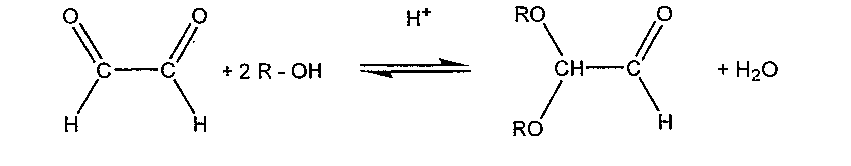

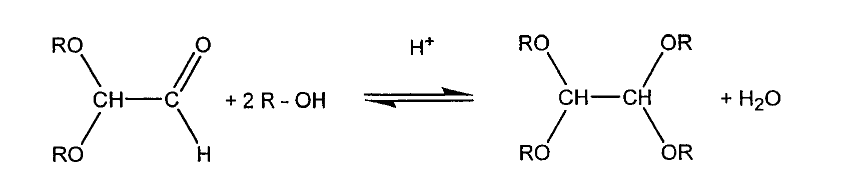

Cette réaction est équilibrée et conduit à un mélange contenant du glyoxal, de l'alcool R-OH, de l'eau, du monoacétal de glyoxal et du diacétal de glyoxal.

La réaction d'acétalisation est une réaction complexe, au cours de laquelle se forment de nombreux oligomères et/ou des produits secondaires cycliques, à côté du monoacétal et du diacétal (voir par exemple J.M. Kliegman et al. dans J. Org. Chem. Vol.38 (1973) p. 556-560 ; A. Stambouli et al., Bull. Soc. Chimique France (1983) II p. 33-44).

Selon l'art antérieur, les procédés de fabrication de diacétals du glyoxal présentent l'inconvénient d'un faible rendement en diacétal, c'est-à-dire en 1,1,2,2-tétraalcoxyéthane, comme décrit par exemple dans le brevet US 2 360 959 (38 % pour le tétraméthoxyéthane), ou nécessitent l'élimination de l'eau réactionnelle par utilisation d'un solvant azéotropant (par exemple dans le brevet GB 559 362) entraînant des coûts énergétiques et techniques importants.

La demande EP 1 300 383 décrit un procédé de préparation de diacétals du glyoxal par réaction de glyoxal aqueux de 40 à 75 % en poids avec des alcools monovalents dans lequel il n'est pas nécessaire de séparer par distillation l'eau formée pendant la réaction d'acétalisation.

La réaction d'acétalisation du glyoxal avec un alcool est décrite notamment dans les demandes EP 0 607 772 et 0 847 976. Dans la demande EP 0 847 976, la purification du diacétal de glyoxal co-produit lors de la réaction est réalisée par une première distillation de l'alcool en excès, suivie d'une distillation d'un azéotrope contenant de l'eau et le diacétal de glyoxal nécessitant un ajout d'eau et enfin la distillation azéotropique de l'eau avec un tiers solvant azéotropant et la récupération du diacétal de glyoxal anhydre en pied de colonne de distillation.

Cependant, les procédés décrits dans l'art antérieur ne permettent pas de réaliser de manière aisée et quantitative l'étape de séparation du diacétal du glyoxal directement à partir du mélange réactionnel le contenant ainsi que les autres réactifs de la réaction d'acétalisation, à savoir principalement le glyoxal, le monoacétal de glyoxal, l'alcool et l'eau.

Le problème technique à résoudre consiste donc à séparer sélectivement le diacétal du glyoxal du mélange obtenu à l'issue de la réaction d'acétalisation, de manière à obtenir ledit diacétal avec un rendement élevé, et ceci au moyen d'un procédé pouvant être réalisé aussi bien en mode discontinu qu'en mode continu, afin d'optimiser les coûts de production.

On a maintenant trouvé qu'une telle séparation pouvait être effectuée par une extraction liquide-liquide sélective à contre-courant du diacétal de glyoxal à partir du mélange obtenu à l'issue de la réaction d'acétalisation et de l'élimination de l'alcool en excès, ci-après dénommé "mélange brut", à l'aide d'un solvant d'extraction non miscible au milieu réactionnel.

De manière avantageuse, ce procédé de séparation permet d'obtenir le diacétal de glyoxal avec un rendement quasi quantitatif, et notamment de récupérer au moins 95 %, voire au moins 98 % de la quantité de diacétal de glyoxal présente dans le mélange brut.

L'invention concerne donc un procédé de séparation d'un diacétal du glyoxal de formule (I) 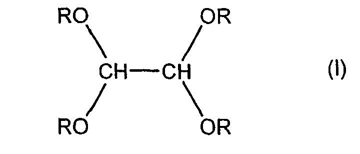 dans laquelle R représente un groupe alkyle en C₁ - C₄ linéaire ou ramifié , à partir d'un mélange aqueux contenant ledit diacétal du glyoxal ainsi qu'un monoacétal du glyoxal de formule (II) 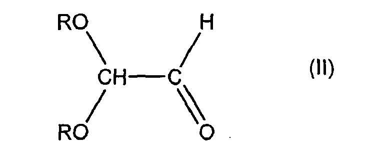 dans laquelle R est tel que défini ci-dessus, au moyen d'au moins une étape d'extraction liquide-liquide à contre-courant dudit diacétal du glyoxal à l'aide d'un solvant non miscible au milieu réactionnel choisi parmi les éthers, les alcanes et les hydrocarbures aromatiques, afin d'obtenir, d'une part, une phase légère contenant ledit diacétal du glyoxal, et, d'autre part, une phase lourde renfermant les autres constituants du mélange brut.

On entend par « solvant non miscible au milieu réactionnel » un solvant qui ne forme pas un mélange homogène avec le milieu réactionnel.

Dans la suite de la description, il est entendu que le monoacétal du glyoxal de formule (II) ci-dessus peut exister également sous sa forme hydratée de formule suivante :

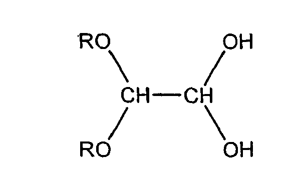

Par "groupe alkyle linéaire ou ramifié en C₁ - C₄ ", on entend en particulier un groupe méthyle, éthyle, propyle, isopropyle ou butyle.

Le mélange brut à partir duquel sera séparé le diacétal du glyoxal de formule (I) comprend majoritairement ledit diacétal, un monoacétal de formule (II), et de l'eau.

Selon un aspect préféré du procédé, le substituant R dans les formules (I) et (II) ci-dessus représente un groupe alkyle en C₁-C₂, et en particulier un groupe méthyle.

Lorsque R représente le méthyle, le diacétal de glyoxal de formule (I) est le tétraméthoxy 1,1,2,2-éthane (TME) et le monoacétal de formule (II) est le diméthoxyéthanal (DME).

Dans ce cas, le mélange brut comprend notamment, en pourcentages massiques, 25 à 60 % de TME, 7 à 35 % de DME et 20 à 50 % d'eau. Ledit mélange peut également comprendre, en pourcentages massiques, 0 à 15 % de glyoxal, 0 à 10 % de méthanol et 0 à 5 % d'impuretés. Les impuretés sont essentiellement constituées d'éthylène glycol, d'acétaldéhyde ou des produits secondaires de la réaction d'acétalisation tels que les acétals cycliques, les oligomères de glyoxal, les oligomères de DME etc...

Le choix du solvant d'extraction représente un aspect clé du procédé.

En effet, de manière surprenante, on s'est aperçu que le diacétal du glyoxal, et en particulier le TME, a un comportement de partage très différent du monoacétal du glyoxal correspondant (à savoir, le DME dans le cas du TME) vis-à-vis du solvant, lorsque les deux composés précités se trouvent en phase aqueuse. De plus le partage du diacétal du glyoxal, et en particulier du TME, vers le solvant est très différent lorsqu'il se trouve seul en phase aqueuse et lorsqu'il se trouve en mélange avec le monoacétal du glyoxal correspondant (à savoir le DME dans le cas du TME).

De préférence, ledit solvant est choisi parmi les éthers, les alcanes et les hydrocarbures aromatiques.

On peut citer à titre d'exemple l'éther disopropylique, le méthyl tert-butyl éther (MTBE), le méthyl teramyl éther (TAME), le n-hexane, le méthylcyclohexane, le xylène.

Des solvants particulièrement avantageux aux fins de l'invention sont le cyclohexane, le n-heptane et le toluène.

L'étape d'extraction liquide-liquide à contre courant peut être réalisée à l'aide d'une ou plusieurs colonnes d'extraction, en série ou en parallèle, telles que celles décrites notamment dans l'ouvrage de référence « Perry's Chemical Engineers' Handbook, 7th Edition , Mc Graw Hill Ed., année 1997, Chapitre 15, p. 15-28 à 15-31.

On utilisera de préférence une combinaison de plusieurs mélangeurs-décanteurs. A titre d'exemple, dans la catégorie des extracteurs fonctionnant par gravité sans agitation mécanique, on peut utiliser une colonne à pulvérisation , une colonne à garnissage ou une colonne à plateaux perforés. Parmi les extracteurs fonctionnant par gravité avec agitation mécanique, on peut utiliser, par exemple, une colonne à agitateur tournant ou une colonne pulsée.

On peut également utiliser un extracteur centrifuge.

On utilisera de préférence aux fins de l'invention une colonne avec agitateur tournant.

Selon le procédé de séparation de l'invention, le mélange brut est introduit sur la colonne d'extraction liquide-liquide et extrait à l'aide d'un flux à contre courant de solvant d'extraction.

La phase légère contenant le diacétal de glyoxal de formule (I) et le solvant d'extraction est recueillie en tête de colonne. Avantageusement, cette phase légère contient au moins 95 %, de préférence au moins 98 % de la quantité de diacétal de glyoxal de formule (1) initialement présente dans le mélange brut et moins de 10 %, de préférence moins de 1 % de la quantité de monoacétal de glyoxal de formule (II) initialement présente dans le mélange brut.

A cet effet, la quantité de solvant est de préférence adaptée de manière à extraire au moins 95 % du diacétal de glyoxal de formule (I) tout en restant minimale. On utilisera de préférence un rapport massique solvant / mélange brut compris entre 0,3/1 et 5/1, la quantité optimale de solvant étant adaptée en fonction du solvant et de l'appareillage utilisés.

L'extraction est réalisée à une température inférieure, égale ou supérieure à la température ambiante, en restant inférieure à la température d'ébullition du solvant. De préférence, l'extraction est réalisée à une température de 10°C à 60°C, de préférence à température ambiante.

La phase légère obtenue à l'issue de l'extraction est soumise à une séparation, par exemple par distillation sous pression réduite, à l'issue de laquelle on récupère, d'une part, ledit diacétal de glyoxal et, d'autre part, le solvant d'extraction qui peut être recyclé à l'étape d'extraction liquide-liquide.

De préférence, cette distillation est effectuée sous pression réduite, en particulier à une pression de 200 à 300 mbar. La température du pied de distillation est de préférence inférieure ou égale à 120°C.

La phase lourde contenant majoritairement de l'eau et du monoacétal de glyoxal est recueillie en pied de colonne et concentrée par évaporation à l'issue de laquelle on récupère le monoacétal de glyoxal, le glyoxal résiduel ainsi que le diacétal de glyoxal résiduel, en vue de leur recyclage.

Le mélange brut est obtenu à l'issue de la réaction d'acétalisation représentée plus haut.

On utilisera de préférence un glyoxal aqueux de concentration comprise entre 40 et 75 % en poids, de préférence 60 à 70 % en poids, celui-ci étant obtenu par concentration sous pression réduite de glyoxal aqueux commercial dont la concentration est généralement de l'ordre de 40 %, le glyoxal aqueux sera de préférence concentré immédiatement avant l'utilisation.

Les conditions expérimentales pour la concentration du glyoxal ainsi que l'appareillage sont connus de l'homme du métier et l'on peut citer à titre d'exemple la demande EP-A-1300383 ou R.K . Shah et A.C. Mueller, Heat exchange, Ullmann's Encyclopedia of Industrial Chemistry, 6ème édition sur CD-Rom, Wiley VCH.

Le rapport molaire alcool R-OH / glyoxal sera de préférence compris entre 10/1 et 50/1, de préférence de 10/1 à 30/1, en particulier de 15/1.

On utilisera de préférence les alcools miscibles à l'eau comme le méthanol, l'éthanol, le n- et l'isopropanol. Le mélange brut comprenant du TME et du DME est obtenu par acétalisation du glyoxal avec du méthanol.

En tant que catalyseurs acides, on peut utiliser aussi bien des acides de Lewis que des acides de Brönstedt. On peut utiliser comme catalyseurs ceux qui se dissolvent de façon homogène dans le mélange, tels que par exemple les acides sulfuriques ou les acides sulfoniques organiques comme l'acide méthanesulfonique ou p-toluènesulfonique. On peut également utiliser des catalyseurs hétérogènes comme par exemple le sulfate de zirconium ainsi que des échangeurs d'ions fortement acides, en particulier sulfoniques. Les résines échangeuse d'ions commercialisées sous les marques Lewatit^{®}, BayKat^{®}. (Bayer AG, Leverkusen), Amberlite^{®} et Amberlyst^{®} (Rohm et Haas) ainsi que Dowex^{®} (Dow Chemicals) sont des exemples d'échangeurs d'ions appropriés. Les Lewatit^{®}S100, Lewatit^{®}K2431, Lewatit^{®}K2621, Lewatit^{®}K2629, BayKat^{®}K2611, Amberlyst^{®}15, Amberlyst^{®}35 ainsi que Dowex^{®}50 sont des échangeurs d'ions fortement acides également disponibles commercialement.

On peut avantageusement utiliser un catalyseur hétérogène sous la forme d'un lit catalytique fixe, par lequel on introduit le mélange liquide comprenant l'alcool et le glyoxal aqueux. Le mélange liquide peut passer une ou plusieurs fois sur le lit catalytique fixe, jusqu'à ce que le temps de contact ou temps de séjour désiré soit obtenu.

La température utilisée pour l'acétalisation se situe en principe au dessus de la température ambiante et de préférence la réaction est effectuée à une température de 60°C à 140°C, de préférence d'environ 80°C à 130°C, et plus préférentiellement de 100°C à 130°C. De préférence, la réaction est réalisée à une pression supérieure ou égale à la pression atmosphérique, de préférence à une pression supérieure ou égale à la pression atmosphérique et inférieure ou égale à 15 bars.

La réaction du glyoxal avec l'alcool est conduite jusqu'à atteindre approximativement l'état d'équilibre, c'est à dire jusqu'à ce que la concentration en diacétal dans le milieu réactionnel s'établisse au moins à 70 %, de préférence à au moins 80 %, plus particulièrement au moins à 90 % et encore plus préférentiellement au moins à 95 % de la concentration à l'équilibre.

Selon un aspect avantageux du procédé selon l'invention, le mélange brut est obtenu à l'issue d'une réaction d'acétalisation effectuée pendant une durée inférieure ou égale à 1 h, de préférence inférieure ou égale à 40 min, en particulier inférieure ou égale à 20 min.

Après avoir atteint l'équilibre réactionnel, le mélange réactionnel est neutralisé puis est distillé , de préférence à pression atmosphérique avec une température de pied inférieure à 110°C, pour éliminer l'alcool en excès qui est recyclé au niveau de la colonne d'acétalisation.

Selon un aspect avantageux de l'invention, la réaction d'acétalisation, l'étape d'extraction liquide-liquide et la récupération des différents constituants du mélange brut peuvent être réalisées en mode continu. Dans ce cas, le glyoxal, le monoacétal du glyoxal, l'alcool R-OH et le solvant d'extraction sont recyclés.

A cet effet, le glyoxal et le monoacétal du glyoxal résultant de la concentration de la phase lourde sont réintroduits dans le circuit d'alimentation de la colonne dans laquelle est effectuée l'acétalisation. Alternativement, la phase aqueuse lourde avant concentration peut directement alimenter l'évaporateur servant à la concentration du glyoxal.

Le solvant d'extraction résultant de la séparation de la phase légère, en particulier par distillation, est avantageusement récupéré en quasi totalité et réintroduit dans l'alimentation de la colonne d'extraction liquide-liquide.

L'invention est illustrée par les exemples ci-dessous.

### Exemple 1 :

Dans une colonne R1, équipée d'une double enveloppe, de 12 cm de diamètre interne et de 130 cm de hauteur , thermostatée à 68 +/- 2°C par une circulation d'eau chaude dans l'enceinte extérieure et contenant 13 L de résine échangeuse de cations à groupements sulfoniques sous forme acide, d'une capacité d'échange de 3 +/- 2 eq/L, on fait circuler, à la pression atmosphérique et à vitesse constante, 785 g/h d'un mélange contenant en proportions massiques, 88 % de méthanol, 8 % de glyoxal et 4 % d'eau, obtenu en ajoutant 686 g/h de méthanol à 89 g/h de glyoxal 70 % en solution aqueuse.

En sortie de colonne, la solution réactionnelle neutralisée contenant pondéralement 74 % de méthanol, 13 % de TME, 8 % d'eau, 4,5 % de DME et moins de 0,5 % de glyoxal non transformé, est distillée en continu sous pression atmosphérique dans une colonne C1 de 20 étages théoriques (DN50), avec un taux de reflux de 2/1, pour récupérer plus de 99 % du méthanol présent que l'on recueille avec moins de 2 % d'eau.

Le méthanol récupéré est recyclé dans l'alimentation de R1. Le pied de distillation (200 +/- 10 g/h) est extrait à température ambiante en continu à contre courant par un flux de 300 +/- 10 g/h de cyclohexane dans une colonne d'extraction liquide-liquide C2 de type Rushton Oldshue d'une trentaine de compartiments agités. En tête de colonne, on recueille 400 +/- 10 g/h de phase légère qui contient plus de 98 % du TME initial et moins de 1 % du DME initial (Cyclohexane 73,5 %, TME 25,1 %, DME 0,5 %, eau 0,9 %). En pied de colonne, on recueille environ 100 +/- 10 g/h de phase lourde qui contient approximativement 58 % d'eau, 36 % de DME, 3,5 % de glyoxal,1 % de TME, 0,8 % de cyclohexane et 0,7 % de méthanol.

La phase légère est distillée sous une pression de 200 mbar en continu sur une colonne C3 (DN 50) de 10 étages théoriques avec un taux de reflux de 1/1 pour récupérer plus de 99 % du cyclohexane présent ainsi que le DME, et obtenir en pied 100 g/h de TME de pureté supérieure à 99 %. La phase lourde d'extraction issue de la colonne C2 est concentrée sur un évaporateur C4 à env. 60°C - 80°C sous une pression de 200 mbar de façon à obtenir 50 +/- 5 g/h d'un mélange contenant 75 % de DME, 17 % d'eau, 7 % de glyoxal et 1% TME qui est mélangé à 275 g/h de méthanol pour être recyclé avec le mélange d'alimentation de la colonne R1.

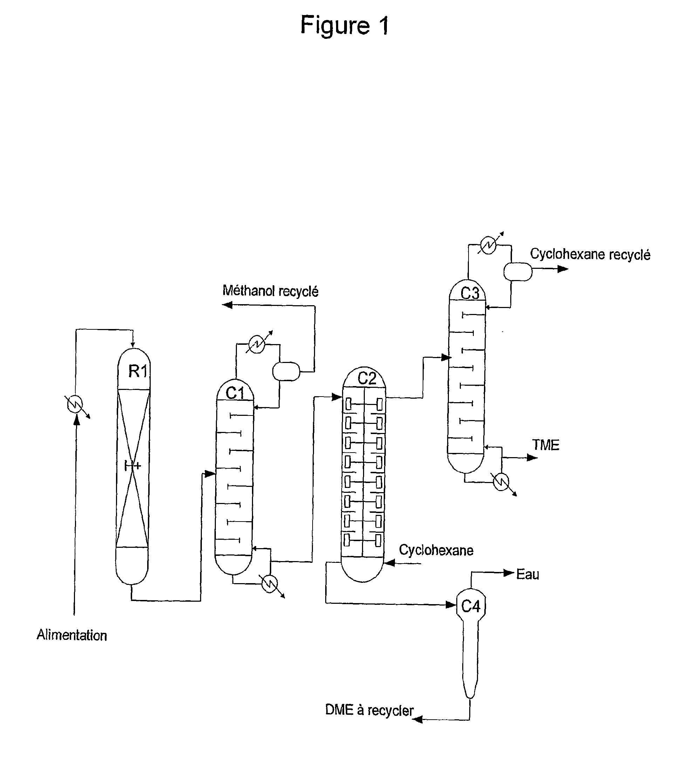

Le procédé réactionnel décrit ci-dessus est représenté sur la figure 1.

### Exemple 2 :

170 g/h d'un mélange contenant 60 % d'eau, 25 % de glyoxal et 15 % de DME sont concentrés sur un évaporateur couche mince de laboratoire E1 sous 100 mbar. La température du fluide thermique circulant dans la double enveloppe est ajustée à 112+/- 2 °C de manière à évaporer 85 +/- 5 g/h d'eau. Le concentrat (85 +/- 5 g/h) contenant 50 % de glyoxal, 30 % de DME et 20 % d'eau est mélangé en ligne avec 615 g/h de méthanol. Ce mélange est alimenté sur une colonne R1, équipée d'une double enveloppe, de 12 cm de diamètre interne et de 130 cm de hauteur, thermostatée à 68 +/- 2°C par une circulation d'eau chaude dans l'enceinte extérieure et contenant 13 L de résine échangeuse de cations à groupements sulfoniques sous forme acide, d'une capacité d'échange de 3 +/- 2 eq/L.

En sortie de colonne, la solution réactionnelle neutralisée contenant pondéralement 74,5 % de Méthanol, 14,5 % de TME, 6,5 % d'Eau, 4 % de DME et moins de 0,3 % de glyoxal non transformé, est distillée en continu sous pression atmosphérique dans une colonne C1 de 20 étages théoriques (DN50), avec un taux de reflux de 2/1, pour récupérer plus de 99 % du méthanol présent que l'on recueille avec moins de 2 % d'eau. Le méthanol récupéré est recyclé en mélange avec le concentrat issu de l'évaporateur E1.

Le pied de distillation (175 +/- 10 g/h) est extrait à température ambiante en continu à contre courant par un flux de 260 g/h de cyclohexane dans une colonne d'extraction liquide-liquide C2 de type Rushton Oldshue d'une trentaine de compartiments agités. En tête de colonne, on recueille 360 +/- 10 g/h de phase légère qui contient plus de 98 % du TME initial et moins de 1 % du DME initial (Cyclohexane 73,5 %, TME 25,1 %, DME 0,5 %, eau 0,9 %). En pied de colonne, on recueille environ 70 +/- 5 g/h de phase lourde qui contient approximativement 59 % d'eau, 38 % de DME et 3 % de glyoxal.

La phase légère est distillée sous une pression de 200 mbar en continu sur une colonne C3 (DN 50) de 10 étages théoriques avec un taux de reflux de 1/1 pour récupérer plus de 99 % du cyclohexane présent ainsi que le DME, et obtenir en pied approximativement 100 g/h de TME de pureté supérieure à 99 %.

La phase lourde d'extraction issue de la colonne C2 est mélangée à 100 g/h d'une solution aqueuse de glyoxal à 40 % de manière à réaliser un mélange contenant 60 % d'eau, 25 % de glyoxal et 15 % de DME qui est utilisé comme alimentation de R1.

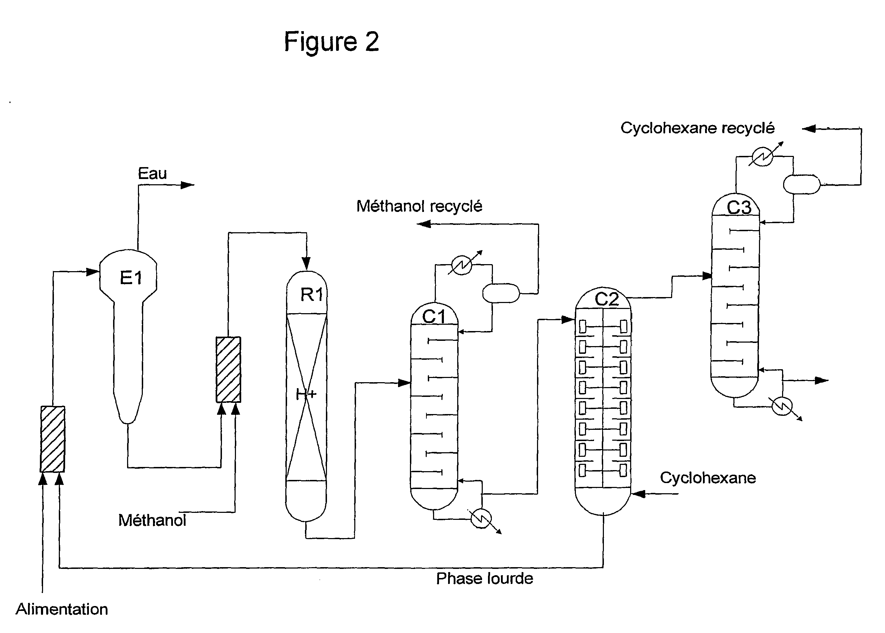

Le procédé réactionnel décrit ci-dessus est représenté sur la figure 2.

### Exemple 3 : préparation du mélange brut

470 g de méthanol et 120 g de glyoxal en solution aqueuse à 70,5 % sont mélangés de façon à obtenir 590 g de solution contenant, en pourcentages massiques, 79,6 % de méthanol, 14,4 % de glyoxal et 6,0 % d'eau, soit un ratio molaire de 10 mol de méthanol par mol de glyoxal (dosé par chromatographie en phase gazeuse).

Cette solution est introduite à température ambiante dans un autoclave ayant pour volume utile 1 L, et étant équipé d'une double enveloppe et d'un système d'agitation, d'une sonde de température et d'un dispositif de prélèvement d'échantillon.

200 g de résine Amberlyst^{®} C35 préalablement rincée au méthanol et séchée sur Büchner sont ajouté au milieu. L'autoclave est ensuite fermé, son ciel gazeux est purgé à l'azote et l'agitation est mise en service à 150 tr.min⁻¹. Le système clos est alors chauffé par circulation de liquide thermostatique à 120°C dans la double enveloppe de l'autoclave. En 20 min, la température interne atteint au moins 95 °C sous une pression d'environ 3 bars. En 30 min, la température interne est d'au moins 105°C sous une pression d'environ 3,8 bars. Après 40 min, le milieu a atteint au moins 90 % de l'équilibre réactionnel. Il contient alors 1,4 % de glyoxal, 15.5 % de TME et 9.5 % de DME.

L'autoclave est refroidi à 25°C par circulation de liquide thermique froid dans la double enveloppe. Le milieu réactionnel est soutiré et filtré sur Büchner de façon à récupérer la résine Amberlyst ® C35 qui peut être réutilisée.

Le mélange obtenu est neutralisé avant d'être purifié par distillation et extraction dans les conditions décrites dans les exemples 1 et 2 ci-dessus, celles-ci étant adaptées en fonction de la quantité de mélange réactionnel.

### Exemple 4 :

Etude du partage du TME entre une phase légère contenant du cyclohexane et une phase lourde contenant de l'eau à 25°C.

50 g de solution aqueuse de TME et DME sont préparés et mis sous agitation à 25°C et pression atmosphérique, dans un ballon de 250 mL. 50 g de solvant sont ajoutés. Le milieu est maintenu sous agitation pendant 30 min puis transféré dans une ampoule à décanter de 250 mL. Après décantation, la phase lourde et la phase légère sont pesées puis analysées par chromatographie.

Les résultats rapportés dans le tableau 1 ci-dessous sont obtenus en extrayant 50 g de solutions aqueuses de TME et DME avec 50 g de cyclohexane.

**Tableau 1**

| | **Phase lourde** | | **Phase légère** | | | |
|---|---|---|---|---|---|---|
| **Essai N°** | **DME** | **TME** | **DME** | **TME** | **Coefficient de partage** | **Coefficient de partage** |
| | **% massique** | **% massique** | **% massique** | **% massique** | **TME** | **DME** |
| **1** | 0 | 20,2 | 0 | 10,8 | 0,5 | |
| **2** | 15,3 | 23,6 | 0,15 | 26,0 | 1,1 | 0,01 |
| **3** | 32,4 | 18,6 | 0,5 | 27,6 | 1,5 | 0,02 |

Les résultats montrent que lorsque la solution aqueuse de TME de départ ne contient pas de DME (essai 1), le coefficient de partage du TME (% massique de TME dans la phase organique / % massique de TME dans la phase aqueuse) est égal à 0,5.

La présence de DME dans la solution aqueuse de départ (essais 2-3) augmente de façon inattendue le coefficient de partage du TME. En effet pour des quantités à peu près stables de TME en phase lourde (18,6 à 23,6 %), le coefficient de partage du TME passe de 0,5 sans DME à 1,1 avec, 15,3 % de DME et à 1,5 avec 32,4 % de DME.

## Revendications

1. Procédé de séparation d'un diacétal du glyoxal de formule (I) 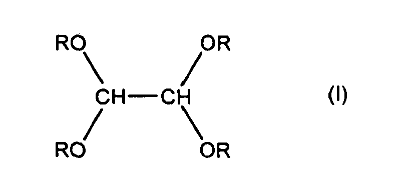 dans laquelle R représente un groupe alkyle en C₁ - C₄ linéaire ou ramifié , à partir d'un mélange brut aqueux comprenant ledit diacétal du glyoxal ainsi qu'un monoacétal du glyoxal de formule (II) 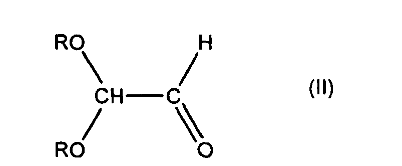 dans laquelle R est tel que défini ci-dessus, **caractérisé en ce qu'**on procède à au moins une étape d'extraction liquide-liquide à contre-courant dudit diacétal du glyoxal à l'aide d'un solvant non miscible au milieu réactionnel, afin d'obtenir, d'une part, une phase légère contenant ledit diacétal du glyoxal, et, d'autre part, une phase lourde renfermant les autres constituants du mélange brut.

2. Procédé selon la revendication 1, **caractérisé en ce que** ledit mélange brut comprend majoritairement un diacétal du glyoxal de formule (I), un monoacétal du glyoxal de formule (II), tels que définis dans la revendication 1, et de l'eau.

3. Procédé selon les revendications 1 ou 2, **caractérisé en ce que** le solvant est choisi parmi les éthers, les alcanes et les hydrocarbures aromatiques.

4. Procédé selon l'une quelconque des revendications 1 à 3, **caractérisé en ce que** le solvant est choisi parmi le cyclohexane, le n-heptane et le toluène.

5. Procédé selon l'une quelconque des revendications 1 à 4, **caractérisé en ce que** le rapport massique solvant / mélange brut est compris entre 0,3/1 et 5/1.

6. Procédé selon l'une quelconque des revendications 1 à 5, **caractérisé en ce que** l'extraction est réalisée à une température de 10°C à 60°C, de préférence à température ambiante.

7. Procédé selon l'une quelconque des revendications 1 à 6, **caractérisé en ce que** la phase légère contenant le diacétal du glyoxal de formule (I) et le solvant est soumise à une séparation à l'issue de laquelle on récupère ledit diacétal du glyoxal.

8. Procédé selon la revendication 7, **caractérisé en ce que** cette séparation est effectuée par distillation sous pression réduite.

9. Procédé selon l'une quelconque des revendications 7 ou 8, **caractérisé en ce que** cette séparation est effectuée à une température comprise entre la température ambiante et 120°C.

10. Procédé selon l'une quelconque des revendications 1 à 9, **caractérisé en ce que** le solvant est recyclé à l'étape d'extraction liquide-liquide.

11. Procédé selon l'une quelconque des revendications 1 à 10, **caractérisé en ce que** le mélange brut est obtenu par une réaction d'acétalisation du glyoxal aqueux de 40 à 75 % en poids avec un alcool de formule R-OH dans lequel R est tel que défini dans la revendication 1, le rapport molaire R-OH/glyoxal étant compris entre 10/1 et 50/1, de préférence 10/1 à 30/1, en présence d'un catalyseur acide, suivie de la distillation du mélange réactionnel obtenu pour éliminer l'alcool R-OH en excès.

12. Procédé selon l'une quelconque des revendications 1 à 11, **caractérisé en ce que** dans les formules (I) et (II) R est un groupe alkyle en C₁-C₂.

13. Procédé selon la revendication 12, **caractérisé en ce que** R est un groupe méthyle.

14. Procédé selon l'une quelconque des revendications 1 à 13, **caractérisé en ce que** l'alcool est le méthanol.

15. Procédé selon l'une quelconque des revendications 1 à 14, **caractérisé en ce que** le mélange brut contient majoritairement du tétraméthoxyl ,1,2,2-éthane (TME), du diméthoxyéthanal (DME) et de l'eau.

16. Procédé selon l'une quelconque des revendications 1 à 15, **caractérisé en ce que** ledit mélange comprend, en pourcentages massiques, 25 à 60 % de TME, 7 à 35 % de DME et 20 à 50 % d'eau.

17. Procédé selon l'une quelconque des revendications 1 à 16, **caractérisé en ce que** ledit mélange comprend également, en pourcentages massiques, de 0 à 15 % de glyoxal, de 0 à 10 % de méthanol et de 0 à 5 % d'impuretés.

18. Procédé selon l'une quelconque des revendications 11 à 17, **caractérisé en ce que** le glyoxal utilisé dans la réaction d'acétalisation est concentré à 60 à 70 %.

19. Procédé selon la revendication 18, **caractérisé en ce que** le glyoxal est concentré à partir d'une solution aqueuse.

20. Procédé selon l'une quelconque des revendications 11 à 19, **caractérisé en ce que** la réaction d'acétalisation est effectuée pendant une durée inférieure ou égale à 1 h, de préférence inférieure ou égale à 40 min.

21. Procédé selon la revendication 20, **caractérisé en ce que** la durée de la réaction est inférieure ou égale à 20 min.

22. Procédé selon l'une des revendications 11 à 21, **caractérisé en ce que** la réaction d'acétalisation est effectuée à une température de 60°C à 140°C, de préférence de 80°C à 130°C.

23. Procédé selon la revendication 22, **caractérisé en ce que** la température est de 100 à 130°C.

24. Procédé selon l'une des revendications 11 à 23, **caractérisé en ce que** la réaction d'acétalisation est effectuée à une pression supérieure ou égale à la pression atmosphérique.

25. Procédé selon la revendication 24, **caractérisé en ce que** la pression est inférieure ou égale à 15 bars.

26. Procédé selon l'une quelconque des revendications 1 à 25, **caractérisé en ce que** la réaction d'acétalisation, l'étape d'extraction liquide-liquide et la récupération des différents constituants du mélange brut sont réalisées en continu, le glyoxal, le monoacétal du glyoxal, l'alcool R-OH et le solvant d'extraction étant recyclés.

## Claims

1. Process for the separation of a glyoxal diacetal of formula (I) 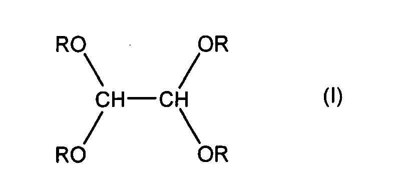 in which R represents a linear or branched C₁ - C₄ alkyl group, from a crude aqueous mixture comprising said glyoxal diacetal and a glyoxal monoacetal of formula (II) 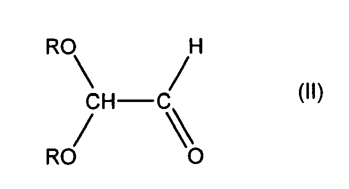 in which R is as defined above, **characterized in that** at least one step of countercurrentwise liquid-liquid extraction of said glyoxal diacetal is carried out using a solvent which is immiscible with the reaction medium, in order to obtain, on the one hand, a light phase comprising said glyoxal diacetal and, on the other hand, a heavy phase including the other constituents of the crude mixture.

2. Process according to Claim 1, **characterized in that** said crude mixture comprises predominantly a glyoxal diacetal of formula (I) as defined in Claim 1, a glyoxal monoacetal of formula (II) as defined in Claim 1, and water.

3. Process according to Claim 1 or Claim 2, **characterized in that** the solvent is chosen from ethers, alkanes and aromatic hydrocarbons.

4. Process according to any one of Claims 1 to 3, **characterized in that** the solvent is chosen from cyclohexane, n-heptane and toluene.

5. Process according to any one of Claims 1 to 4, **characterized in that** the solvent/crude mixture ratio by weight is between 0.3/1 and 5/1.

6. Process according to any one of Claims 1 to 5, **characterized in that** the extraction is carried out at a temperature of 10°C to 60°C, preferably at ambient temperature.

7. Process according to any one of Claims 1 to 6, **characterized in that** the light phase comprising the glyoxal diacetal of formula (I) and the solvent is subjected to a separation, on conclusion of which said glyoxal diacetal is recovered.

8. Process according to Claim 7, **characterized in that** this separation is carried out by distillation under reduced pressure.

9. Process according to either one of Claims 7 and 8, **characterized in that** this separation is carried out at a temperature of between ambient temperature and 120°C.

10. Process according to any one of Claims 1 to 9, **characterized in that** the solvent is recycled to the liquid-liquid extraction step.

11. Process according to any one of Claims 1 to 10, **characterized in that** the crude mixture is obtained by an acetalization reaction of 40 to 75% by weight aqueous glyoxal with an alcohol of formula R-OH in which R is as defined in Claim 1, the R-OH/glyoxal molar ratio being between 10/1 and 50/1, preferably 10/1 to 30/1, in the presence of an acid catalyst, followed by the distillation of the reaction mixture obtained in order to remove the excess alcohol R-OH.

12. Process according to any one of Claims 1 to 11, **characterized in that**, in the formulae (I) and (II), R is a C₁-C₂ alkyl group.

13. Process according to Claim 12, **characterized in that** R is a methyl group.

14. Process according to any one of Claims 1 to 13, **characterized in that** the alcohol is methanol.

15. Process according to any one of Claims 1 to 14, **characterized in that** the crude mixture comprises predominantly 1,1,2,2-tetramethoxy-ethane (TME), dimethoxyethanal (DME) and water.

16. Process according to any one of Claims 1 to 15, **characterized in that** said mixture comprises, as percentages by weight, 25 to 60% of TME, 7 to 35% of DME and 20 to 50% of water.

17. Process according to any one of Claims 1 to 16, **characterized in that** said mixture also comprises, as percentages by weight, 0 to 15% of glyoxal, 0 to 10% of methanol and 0 to 5% of impurities.

18. Process according to any one of Claims 11 to 17, **characterized in that** the glyoxal used in the acetalization reaction is concentrated to 60 to 70%.

19. Process according to Claim 18, **characterized in that** the glyoxal is concentrated from an aqueous solution.

20. Process according to any one of Claims 11 to 19, **characterized in that** the acetalization reaction is carried out for a period of time of less than or equal to 1 h, preferably of less than or equal to 40 min.

21. Process according to Claim 20, **characterized in that** the period of time of the reaction is less than or equal to 20 min.

22. Process according to one of Claims 11 to 21, **characterized in that** the acetalization reaction is carried out at a temperature of 60°C to 140°C, preferably 80°C to 130°C.

23. Process according to Claim 22, **characterized in that** the temperature is of 100 to 130°C.

24. Process according to one of Claims 11 to 23, **characterized in that** the acetalization reaction is carried out at a pressure of greater than or equal to atmospheric pressure.

25. Process according to Claim 24, **characterized in that** the pressure is less than or equal to 15 bar.

26. Process according to any one of Claims 1 to 25, **characterized in that** the acetalization reaction, the liquid-liquid extraction step and the recovery of the various constituents of the crude mixture are carried out continuously, the glyoxal, the glyoxal monoacetal, the alcohol R-OH and the extraction solvent being recycled.

## Patentansprüche

1. Verfahren zur Abtrennung eines Diacetals von Glyoxal der Formel (I) 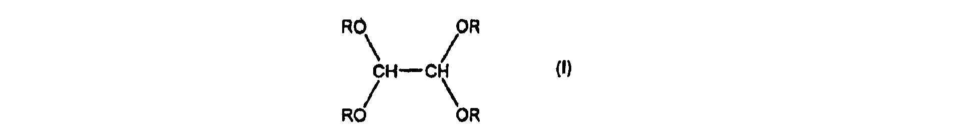 in der R eine lineare oder verzweigte C₁-C₄.Alkylgruppe darstellt,
aus einem wässrigen rohen Gemisch, das das Diacetal von Glyoxal sowie ein Monoacetal von Glyoxal der Formel II 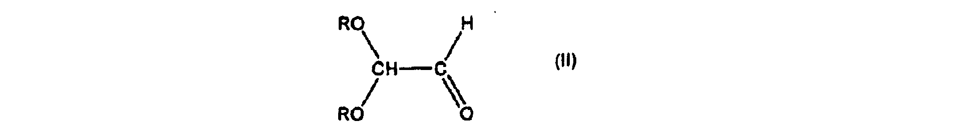 in der R wie oben definiert ist, umfasst, **dadurch gekennzeichnet, dass** man wenigstens eine Stufe der Gegenstrom-Flüssig-Flüssig-Extraktion des Diacetals von Glyoxal mit Hilfe eines Lösungsmittels, das mit dem Reaktionsmedium nicht mischbar ist, durchführt, um einerseits eine leichte Phase, die das Diacetal von Glyoxal enthält, und andererseits eine schwere Phase, die die anderen Bestandteile des rohen Gemisches umfasst, zu erhalten.

2. Verfahren nach Anspruch 1, **dadurch gekennzeichnet, dass** das rohe Gemisch hauptsächlich ein Diacetal von Glyoxal der Formel (I), ein Monoacetal von Glyoxal der Formel (II), wie sie in Anspruch 1 definiert sind, und Wasser umfasst.

3. Verfahren nach Anspruch 1 oder 2, **dadurch gekennzeichnet, dass** das Lösungsmittel unter den Ethern, den Alkanen und den aromatischen Kohlenwasserstoffen ausgewählt wird.

4. Verfahren nach einem der Ansprüche 1 bis 3, **dadurch gekennzeichnet, dass** das Lösungsmittel unter Cyclohexan, n-Heptan und Toluol ausgewählt wird.

5. Verfahren nach einem der Ansprüche 1 bis 4, **dadurch gekennzeichnet, dass** das Masseverhältnis Lösungsmittel/rohes Gemisch zwischen 0,3/1 und 5/1 liegt.

6. Verfahren nach einem der Ansprüche 1 bis 5, **dadurch gekennzeichnet, dass** die Extraktion bei einer Temperatur von 10 °C bis 60 °C, vorzugsweise bei Umgebungstemperatur, durchgeführt wird.

7. Verfahren nach einem der Ansprüche 1 bis 6, **dadurch gekennzeichnet, dass** die leichte Phase, die das Diacetal von Glyoxal der Formel (I) und das Lösungsmittel enthält, einer Trennung unterworfen wird, an deren Ende man das Diacetal von Glyoxal gewinnt.

8. Verfahren nach Anspruch 7, **dadurch gekennzeichnet, dass** diese Trennung durch Destillation unter verringertem Druck durchgeführt wird.

9. Verfahren nach einem der Ansprüche 7 oder 8, **dadurch gekennzeichnet, dass** diese Trennung bei einer Temperatur zwischen der Umgebungstemperatur und 120 °C durchgeführt wird.

10. Verfahren nach einem der Ansprüche 1 bis 9, **dadurch gekennzeichnet, dass** das Lösungsmittel zu der Stufe der Flüssig-Flüssig-Extraktion zurückgeführt wird.

11. Verfahren nach einem der Ansprüche 1 bis 10, **dadurch gekennzeichnet, dass** das rohe Gemisch durch eine Acetalisierungsreaktion von wässrigem Glyoxal mit 40 bis 74 Gew.-% mit einem Alkohol der Formel R-OH, in der R wie in Anspruch 1 definiert ist, wobei das Molverhältnis R-OH/Glyoxal zwischen 10/1 und 50/1 liegt, vorzugsweise 10/1 bis 30/1 ist, in Gegenwart eines Säurekatalysators, gefolgt von der Destillation des erhaltenen Reaktionsgemisches, um den überschüssigen Alkohol R-OH zu entfernen, erhalten wird.

12. Verfahren nach einem der Ansprüche 1 bis 11, **dadurch gekennzeichnet, dass** in den Formeln (I) und (II) R eine C₁-C₂-Alkylgruppe ist.

13. Verfahren nach Anspruch 12, **dadurch gekennzeichnet, dass** R eine Methylgruppe ist.

14. Verfahren nach einem der Ansprüche 1 bis 13, **dadurch gekennzeichnet, dass** der Alkohol Methanol ist.

15. Verfahren nach einem der Ansprüche 1 bis 14, **dadurch gekennzeichnet, dass** das rohe Gemisch hauptsächlich Tetramethoxy-1,1,2,2-ethan (TME), Dimethoxyethanal (DME) und Wasser enthält.

16. Verfahren nach einem der Ansprüche 1 bis 15, **dadurch gekennzeichnet, dass** das Gemisch in Wasser-Prozenten 25 bis 60 % TME, 7 bis 35 % DME und 20 bis 50 % Wasser umfasst.

17. Verfahren nach einem der Ansprüche 1 bis 16, **dadurch gekennzeichnet, dass** das Gemisch in Masse-Prozenten auch 0 bis 15 % Glyoxal, 0 bis 10 % Methanol und 0 bis 5 % Verunreinigungen umfasst.

18. Verfahren nach einem der Ansprüche 11 bis 17, **dadurch gekennzeichnet, dass** das Glyoxal, das in der Acetalisierungsreaktion verwendet wird, auf 60 bis 70 % konzentriert ist.

19. Verfahren nach Anspruch 18, **dadurch gekennzeichnet, dass** das Glyoxal aus einer wässrigen Lösung konzentriert wird.

20. Verfahren nach einem der Ansprüche 11 bis 19, **dadurch gekennzeichnet, dass** die Acetalisierungsreaktion während einer Dauer von unter oder gleich 1 h, vorzugsweise unter oder gleich 40 min, durchgeführt wird.

21. Verfahren nach Anspruch 20, **dadurch gekennzeichnet, dass** die Dauer der Reaktion unter oder gleich 20 min ist.

22. Verfahren nach einem der Ansprüche 11 bis 21, **dadurch gekennzeichnet, dass** die Acetalisierungsreaktion bei einer Temperatur von 60 °C bis 140 °C, vorzugsweise von 80 °C bis 130 °C, durchgeführt wird.

23. Verfahren nach Anspruch 22, **dadurch gekennzeichnet, dass** die Temperatur 100 bis 130 °C ist.

24. Verfahren nach einem der Ansprüche 11 bis 23, **dadurch gekennzeichnet, dass** die Acetalisierungsreaktion bei einem Druck durchgeführt wird, der über Atmosphärendruck oder gleich Atmosphärendruck ist.

25. Verfahren nach Anspruch 24, **dadurch gekennzeichnet, dass** der Druck unter oder gleich 15 bar ist.

26. Verfahren nach einem der Ansprüche 1 bis 25, **dadurch gekennzeichnet, dass** die Acetalisierungsreaktion, die Stufe der Flüssig-Flüssig-Extraktion und die Isolierung der verschiedenen Bestandteile des rohen Gemisches kontinuierlich durchgeführt werden, wobei das Glyoxal, das Monoacetal von Glyoxal, der Alkohol R-OH und das Extraktionslösungsmittel recycelt werden.
